# EUROPEAN PATENT APPLICATION

(11) **EP 2 465 532 A1**
(43) Date of publication of application: **20.06.2012**
(21) Application number: 12159167.1
(22) Date of filing: 31.08.2007
(51) Int. Cl.: A61K 39/12

(54) **JC virus vaccine**

(30) Priority: 31.08.2006 US 841393 P
(62) Divisional of application: 07872285.7
(71) Applicant: Baylor Research Institute, Dallas, TX 75204 (US)
(72) Inventor: Boland, Clement Richard, Dallas, TX 75204 (US)
(74) Representative: Sonn & Partner Patentanwälte

(57) **Abstract**

The present invention includes compositions and methods for the development and use of a vaccine that includes one or more JC virus antigens in a carrier adapted to trigger a JC virus-specific immune response in the human intestinal tract.

SEQUENCE LISTING

<110>  Baylor Research Institute

<120>  JC VIRUS VACCINE

<130>  R61387

<140>  UNKNOWN
<141>  2007-08-31

<150>  US 60/841,393
<151>  2006-08-31

<160>  3

<170>  PatentIn version 3.5

<210>  1
<211>  20
<212>  DNA
<213>  Artificial primers

<220>
<223>  Artificial primer

<400>  1
aatagtggtt taccttaaag                                                    20

<210>  2
<211>  20
<212>  DNA
<213>  Artificial primer

<220>
<223>  Artificial primer

<400>  2
tgaataggga ggaatccatg                                                    20

<210>  3
<211>  23
<212>  DNA
<213>  Artificial Primers

<220>
<223>  Artificial primer

<400>  3
ctgcatgggg gaacattcct gtc                                                23

## Description

### TECHNICAL FIELD OF THE INVENTION

The present relates to vaccination against human polyoma viruses, and more particularly, compositions and methods for the therapeutic use of JC virus and portions thereof to vaccinate patients.

### BACKGROUND OF THE INVENTION

Without limiting the scope of the invention, its background is described in connection with antiviral vaccines.

The JC virus (JCV) is a human polyoma virus that is the etiologic agent of the fatal brain demyelinating disease, progressive multifocal leukoencephalopathy (PML). JCV causes a subacute demyelinizing disease of the brain by a lytic infection of myelin-forming oligodendrocytes and an abortive infection of astrocytes. PML exhibits demyelinizing foci in the cerebrum cerebellum and brain stem and usually ends lethally within a few months.

JCV is present in about 80% of the adult population, however, PML generally only develops in connection with the weakening of the immune system. The increasing use of immunosuppressive drugs and in HIV-infected patients has led to a considerable increase in PML diseases in recent years. According to some estimates, PML develops in about 2-5% of AIDS patients.

Similar to other papovaviruses, the genome of JCV includes three functional domains, the early and the late coding regions separated by the transcriptional control region, which has been shown to be the major determinant of the tropism of this virus for neuroectodermally-derived tissues. The late region encodes capsid proteins produced late in the JCV lytic cycle, while the early region encodes the multifunctional oncoprotein, large tumor antigen (T antigen).

Despite years of study, however, there is no evidence that JCV is associated with colon oncogenesis. Colorectal cancers can show chromosome instability and it has been hypothesized that JCV may account for some of this instability. However, a screen of urine from 45 healthy donors and 233 colorectal cancer/normal tissue pairs for the presence of JCV sequences using the highly sensitive qPCR Taqman assay, found no association. The quantitiative qPCR assay can detect 1 virus genome in 10 human genomes. Despite and infection rate of approximately 70% with four JCV types (2B, 4, 7, and 8) none had a rearranged regulatory region. Among the colon tissues, one normal tissue (<0.5%) and none of the matched tumors tested positive for JCV. Reviewed by, Newcomb, P., et al., "No Evidence of an Association of JC Virus and Colon Neoplasia" Cancer Epidemiol Biomarkers Prev 2004; 13(4), pp. 662-666, April 2004.

### SUMMARY OF THE INVENTION

The present invention includes vaccines, constructions, host cells, and vectors that include or express one or more JC virus antigens for use with a carrier adapted to trigger a JC virus-specific immune response in the human intestinal tract. The one or more JC virus antigens may be obtained from a virus that has been heat-killed, attenuated, chemically-inactivated, mechanically inactivated or combinations thereof and may come from one or more strains of JC virus. The one or more JC virus antigens may include one or more recombinant or synthetically manufactured proteins, synthetic peptides or fragments of JC virus. In one example, the one or more JC virus antigens are produced expressed in a cell from an expression vector that comprises one or more genes or gene fragments from one or more strains of the JC virus. In another example, the one or more JC virus antigens are selected to trigger a cytotoxic T-cell immune response, a humoral immune response or a combination thereof.

For example, the vaccine may include one or more JC virus genes that encode antigens (e.g., complete proteins, fragments of proteins and/or fusion proteins) that are inserted for expression in a carrier virus. When the one or more JC virus antigens are inserted as gene or gene fragments, these may be expressed by a carrier virus, e.g., an attenuated poliovirus. For ease or use, the carrier for the vaccine may be one or more of the well-known pharmaceutically acceptable salt(s). The carrier may also be an adjuvant selected from CFA, IFA, alum, a carrier virus, high molecular weight polysaccharides, glycoproteins and combinations thereof.

The vaccine that includes the one or more JC virus antigens and the carrier may be lyophilized, vacuum-dried, vacuum heat-dried, freeze-sprayed or combinations thereof for, e.g., easy of use, stability and/or storage. The carrier may include one or more excipients, adjuvants, absorption enhancers, release-rate controlling polymer(s), stability enhancers, or combinations thereof. Yet another carrier for either the antigen itself and/or the gene, gene fragment or fusion protein that is used to express and/or deliver the JC virus antigens may be a carrier selected from, e.g., an attenuated polio virus selected from the group consisting of polio virus type 1, polio virus type 2, polio virus type 3, and mixtures thereof, comprising one or more JC virus genes. If using an inactivated polio virus, it may be selected from the group consisting of polio virus type 1, polio virus type 2, polio virus type 3, and mixtures thereof, comprising one or more JC virus genes. Alternatively, other carrier for use with the present invention include an attenuated poliovirus, inactivated poliovirus, hepatitis A virus, echovirus, rhinovirus and coxsackievirus, that express one or more JC virus genes.

The present invention also includes a method for vaccinating a mammal against JC Virus by providing the mammal with one or more JC virus antigens in a carrier adapted to trigger a JC virus-specific immune response in the mammal's intestinal tract. The one or more JC virus antigens may be obtained from one or more strains of JC virus that has been heat-killed, attenuated, chemically-inactivated, mechanically inactivated or combinations thereof. The vaccine may include one or more JC virus antigens comprise one or more recombinant proteins, synthetic peptides or fragments of JC virus.

### BRIEF DESCRIPTION OF THE DRAWINGS

For a more complete understanding of the features and advantages of the present invention, reference is now made to the detailed description of the invention along with the accompanying figures and in which:

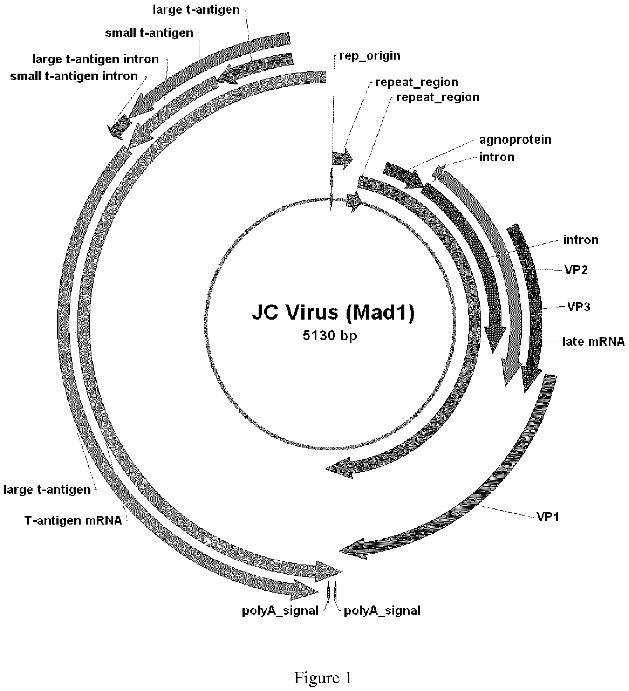

Figure 1 is a map of the Mad1 JCV.

### DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

A number of vaccines have a short shelf life and must be stored at refrigeration temperatures. Optimally, a vaccine should have a long shelf life when stored at room temperatures, however, live vaccines tend to require storage at cold temperatures (even when the vaccine is lyophilized), due to the fact that the number of viable vaccine units drops with prolonged storage at warmer temperatures. While killed or dead vaccines are more stable than live vaccines, live attenuated vaccines are more often used for intestinal vaccination due to the long-term, residual immunity that they provide and the low infectivity of the vaccine.

In general, only a few vaccines are administered orally, the only commonly used oral vaccine is the attenuated polio virus. While the attenuate virus may be killed by acid conditions in the stomach, the vaccine has been formulated in a manner that sufficient viable virus particles pass through the stomach to be active in the small intestine.

As used herein, the term "antigen" refers to a molecule with one or more epitopes that stimulate a host's immune system to make a secretory, humoral and/or cellular antigen-specific response, or to a DNA molecule that is capable of producing such an antigen in a vertebrate. The term is also used interchangeably with "immunogen." For example, a specific antigen can be complete protein, portions of a protein, peptides, fusion proteins, glycosylated proteins and combinations thereof. For use with the presen invention, one or more JCV antigens (native protein or protein fragment), may be provided directly or as part of a recombinant nucleic acid expression system to provide an antigenic JCV product to trigger a host immune response. The JCV antigen may further be a DNA molecule which produces the JCV antigen in the host.

As used herein, the term "gene" refers to a functional protein, polypeptide or peptide-encoding nucleic acid unit. As will be understood by those in the art, this functional term includes genomic sequences, cDNA sequences, or fragments or combinations thereof, as well as gene products, including those that may have been designed and/or altered by the user. Purified genes, nucleic acids, protein and the like are used to refer to these entities when identified and separated from at least one contaminating nucleic acid or protein with which it is ordinarily associated.

As used herein, the term "host cell" refers to cells that have been engineered to contain nucleic acid segments or altered segments, whether archeal, prokaryotic, or eukaryotic. Thus, engineered, or recombinant cells, are distinguishable from naturally occurring cells that do not have the recombinantly introduced genes.

As used herein, the expressions "cell" and "cell culture" are used interchangeably end all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Different designations are will be clear from the contextually clear.

As used herein, the term "plasmids" refers to extrachromosomal, at least partially self-replicating nucleic acids. Plasmids are designated by a lower case p preceded and/or followed by capital letters and/or numbers that name the plasmid. Many plasmids are commercially available, are publicly available on an unrestricted basis, or can be constructed from such available plasmids in accord with published procedures. In addition, other equivalent plasmids are known in the art and will be apparent to the ordinary artisan.

As used herein, the term "protein-protein complex" or "protein complex" refers to an association of more than one protein. The proteins of the complex may be associated by a variety of methods, or by any combination of methods, including but not limited to functional, stereochemical, conformational, biochemical, or electrostatic association. It is intended that the term encompass associations of any number of proteins.

As used herein, the terms "protein", "polypeptide" and "peptide" refer to compounds comprising amino acids joined via peptide bonds and are used interchangeably.

As used herein, the term "transformation," refers to a process by which exogenous DNA enters and changes a recipient cell. It may occur under natural or artificial conditions using various methods well known in the art. Transformation may rely on any known method for the insertion of foreign nucleic acid sequences into a prokaryotic or eukaryotic host cell. The method is selected based on the host cell being transformed and may include, but is not limited to, viral infection, electroporation, lipofection, and particle bombardment. Such "transformed" cells include stably transformed cells in which the inserted DNA is capable of replication either as an autonomously replicating plasmid or as part of the host chromosome.

As used herein, the term "transfection" refers to the introduction of foreign DNA into eukaryotic cells. Transfection may be accomplished by a variety of means known to the art including, e.g., calcium phosphate-DNA co-precipitation, DEAE-dextran-mediated transfection, polybrene-mediated transfection, electroporation, microinjection, liposome fusion, lipofection, protoplast fusion, retroviral infection, and biolistics. Thus, the term "stable transfection" or "stably transfected" refers to the introduction and integration of foreign DNA into the genome of the transfected cell. The term "stable transfectant" refers to a cell which has stably integrated foreign DNA into the genomic DNA. The term also encompasses cells which transiently express the inserted DNA or RNA for limited periods of time. Thus, the term "transient transfection" or "transiently transfected" refers to the introduction of foreign DNA into a cell where the foreign DNA fails to integrate into the genome of the transfected cell. The foreign DNA persists in the nucleus of the transfected cell for several days. During this time the foreign DNA is subject to the regulatory controls that govern the expression of endogenous genes in the chromosomes. The term "transient transfectant" refers to cells which have taken up foreign DNA but have failed to integrate this DNA.

As used herein, the term "selectable marker" refers to the use of a gene that encodes an enzymatic activity and which confers the ability to grow in medium lacking what would otherwise be an essential nutrient (e.g., the HIS3 gene in yeast cells); in addition, a selectable marker may confer resistance to an antibiotic or drug upon the cell in which the selectable marker is expressed. A review of the use of selectable markers in mammalian cell lines is provided in Sambrook, J., et al., MOLECULAR CLONING: A LABORATORY MANUAL, 2nd ed., Cold Spring Harbor Laboratory Press, New York (1989) pp.16.9-16.15.

As used herein, the term "reporter gene" refers to a gene that is expressed in a cell upon satisfaction of one or more contingencies and which, upon expression, confers a detectable phenotype to the cell to indicate that the contingencies for expression have been satisfied. For example, the gene for Luciferase confers a luminescent phenotype to a cell when the gene is expressed inside the cell. In the present invention, the gene for Luciferase may be used as a reporter gene such that the gene is only expressed upon the splicing out of an intron in response to an effector. Those cells in which the effector activates splicing of the intron will express Luciferase and will glow.

As used herein, the term "vector" is used in reference to nucleic acid molecules that transfer DNA segment(s) from one cell to another. The term "vehicle" is sometimes used interchangeably with "vector." The term "vector" as used herein also includes expression vectors in reference to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

As used herein, the term "amplify", when used in reference to nucleic acids refers to the production of a large number of copies of a nucleic acid sequence by any method known in the art. Amplification is a special case of nucleic acid replication involving template specificity. Template specificity is frequently described in terms of "target" specificity. Target sequences are "targets" in the sense that they are sought to be sorted out from other nucleic acid. Amplification techniques have been designed primarily for this sorting out.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (i.e., in the presence of nucleotides and an inducing agent such as DNA polymerase and at a suitable temperature and pH). The primer may be single stranded for maximum efficiency in amplification but may alternatively be double stranded. If double stranded, the primer is first treated to separate its strands before being used to prepare extension products. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (i.e., a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (e.g. ELISA, as well as enzyme-based histochemical assays), fluorescent, radioactive, and luminescent systems. It is not intended that the present invention be limited to any particular detection system or label.

As used herein, the term "target" when used in reference to the polymerase chain reaction, refers to the region of nucleic acid bounded by the primers used for polymerase chain reaction. Thus, the "target" is sought to be sorted oat from other nucleic acid sequences. A "segment" is defined as a region of nucleic acid within the target sequence.

As used herein, the term "polymerase chain reaction" ("PCR") refers to the method of K.B. Mullis U.S. Patent Nos. 4,683,195, 4,683,202, and 4,965,188, hereby incorporated by reference, which describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (i.e., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified". With PCR, it is possible to amplify a single copy of a specific target sequence in genomic DNA to a level detectable by several different methodologies (e.g., hybridization with a labeled probe; incorporation of biotinylated primers followed by avidin-enzyme conjugate detection; incorporation of 32P-labeled deoxynucleotide triphosphates, such as DCTP or DATP, into the amplified segment). In addition to genomic DNA, any oligonucleotide sequence can be amplified with the appropriate set of primer molecules. In particular the amplified segments created by the PCR process itself are, themselves, efficient templates for subsequent PCR amplifications.

As used herein, the term "immunological response" refers to a composition or vaccine that includes a JCV antigen that triggers in the host a cellular- and/or antibody-mediated immune response to JCV-derived antigens. Usually, such a response may include antibody production (e.g., in the intestinal tract), B cell proliferation, helper T cells, and/or cytotoxic T cells proliferation and/or an anergic response from regulatory cells directed specifically to JCV antigen or antigens included in the composition or vaccine of interest.

As used herein, the terms "vaccine composition" or "vaccine" refer to an antigen that is used to stimulate the immune system of a mammal, e.g., a human, so that current harm is alleviated, or protection against future harm is provided by an adaptive immune response. An immune response may also be provided passively, by transferring immune protection (e.g., antibodies) from one "immunized" host to the recipient that has not been challenged by the antigen and/or is unable to generate an immune response to the antigen.

As used herein, the term "immunization" refers to the process of inducing a continuing protective level of antibody and/or cellular immune response which is directed against a JCV antigen, either before or after exposure of the host to JCV.

As used herein, the term a "pharmacologic dose" refers to an amount sufficient to gives a desired physiological effect.

For oral therapeutic administration, the JCV antigen(s) may be incorporated with excipients and used in the form of ingestible tablets, buccal tables, troches, capsules, elixirs, suspensions, syrups, wafers, and the like. Such compositions and preparations should contain at least 0.1% of the JCV antigen(s). The percentage of the compositions and preparations may, of course, be varied and may conveniently be between about 2 to about 60% of the weight of the unit. The amount of the JCV antigen(s) are selected and may be increased or decreased, as will be know to those of skill in the art of vaccination, depending on the therapeutically useful results of one or more vaccinations such that a suitable dosage will be obtained that is immunogenic, that is, it triggers an immune response.

The JCV antigen(s) may also be administered parenterally or intraperitoneally. Solutions of the JCV antigen(s) (or vectors that deliver the JCV antigen(s)) may be provided as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable, oral or other use include sterile aqueous solutions or dispersions and sterile powders for

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The JCV antigen(s) may be included for intramuscular, subcutaneous or even for transdermal administration and may include a reservoir adapted to retain during storage and release in operation the particles containing the JCV antigen(s) of the present invention. It will be appreciated that a wide variety of transdermal devices have been described in the art and are suitable for use in the present invention. An exemplary transdermal device generally includes a reservoir defined by an impermeable backing layer and a membrane. The backing layer and the membrane are joined together about the outer periphery of the device. These layers may be joined by an adhesive, a heat seal or the like. The transdermal device may also include an adhesive layer to attach the device to the skin of a subject. A release liner will generally cover the adhesive that the user removes prior to use of the device to expose adhesive layer.

JC virus (JCV) is a polyoma virus that commonly infects humans. The present inventors have previously shown that T antigen DNA sequences of JCV have been found in the mucosa of normal human colons, colorectal cancers, colorectal cancer xenografts raised in nude mice, and in the human colon cancer cell line SW480. A larger number of viral copies are present in cancer cells than in non-neoplastic colon cells, and sequence microheterogeneity occurs within individual colonic mucosal specimens. The improved yield of detection after treatment with topoisomerase I suggests that the viral DNA is negatively supercoiled in the human tissues. These results indicate that JCV DNA can be found in colonic tissues, which raises the possibility that this virus may play a role in the chromosomal instability observed in colorectal carcinogenesis. Luigi, L. et al., "JC virus DNA is present in the mucosa of the human colon and in colorectal cancers," Proc Natl Acad Sci U S A. 1999 June 22; 96(13): 7484-7489.

Polyomaviruses are widespread among animal species, the two best known being the polyomavirus of mice and the SV40 of the African green monkey (5-13). In humans, the two most extensively studied are JCV and the BK virus, which are equally frequent in humans. Both viruses have approximately 70% DNA sequence homology with SV40, whose human cell transforming properties have been widely studied. This observation has led to extensive speculation concerning the oncogenic potentialities of the two human viruses. However, the reports of an association in humans between polyomavirus infection (including SV40) and tumorigenesis have been variable and controversial (28-45).

Human cancers often are characterized by aneuploidy and widespread chromosomal rearrangements that result in the excessive activity of certain growth-stimulating genes and the deletion of other growth-limiting (tumor suppressor) genes. These genomic deletions are the result of an active process called chromosomal instability, which can be detected at the earliest stages of multistage carcinogenesis of colorectal tumors. The mechanism that permits the accumulation of this extreme degree of chromosomal disorder in cancer currently is unexplained. Aneuploid lymphocytes termed rogue cells have been encountered in short-term lymphocyte cultures of people from locations throughout the world.

Epidemiological evidence suggest rogue cells may be the result of infection by the very widespread JC polyoma virus, a DNA virus with a supercoiled 5.13-kb genome that shows a high degree of homology with the well-known, fibroblast-transforming simian virus 40 (SV40). Significant antibody titers to JC virus (JCV) capsid protein are encountered in 70-80% of adult populations throughout the world. Rogue cells are so badly damaged it is unlikely they would often survive mitosis. Consequently, these cells are assumed to exist only transiently. However, the data suggest that there also may be a significant increase in simple chromosome damage in the cultured lymphocytes of people exhibiting rogue cells.

The possible wider significance of the JCV depends on what cells or tissues it can infect in addition to the lymphocytoid series. The virus lytically infects oligodendrocytes, the principal target cells in humans. Its activation from latency in people with immunosuppressive conditions, particularly AIDS, is responsible for the demyelinating central nervous system disorder, progressive multifocal leukoencephalopathy. JCV DNA also has been isolated from an unusual oligoastrocytoma, although no clear association between JCV and glial tumors has been shown. In Japan, viral DNA has been recovered from 45% of urine samples obtained from older persons. The virus is presumed latent in the kidney as well as in lymphoid tissues such as bone marrow (15-18).In this paper, we report the detection of JC viral DNA fragments from a fourth tissue, very different from those in which the virus previously has been reported, namely, normal and malignant colorectal epithelium. That this viral DNA presence is not the result of the occurrence in the tissue of transitory, infected cells of the lymphocytoid line is strongly suggested by the fact that viral nucleotide sequences also were detected in five of 10 colon cancer xenografts and in the colon cancer cell line SW480.

### Example 1. JCV Cloning and Manipulation.

Human Tissue Specimens. Matched pairs of colorectal cancer and normal, adjacent mucosa from surgical resection specimens are obtained. DNA is isolated from each specimen and used as a template for the PCR to amplify DNA sequences coding the amino terminus of the JCV T antigen. Specimens of surgically resected human colorectal tissues from patients with cancer were obtained from the University of Michigan School of Medicine Department of Pathology after surgical resection, under Institution Review Board approval, and after a variable period of ischemic time (usually 30-60 min), were slowly frozen and stored at -80°C. The surgical samples are thawed and homogenized in Trizol (GIBCO) by using a 1.5-ml plastic tube (Kontes) and a disposable pestle for each specimen. Care is taken to prevent carryover between specimens. The DNA-containing fraction are digested overnight in proteinase K (Boehringer Mannheim), followed by extraction with phenol/chloroform, and quantitated by spectrophotometry (OD₂₆₀).

PCR may be used to isolate one or more species of JCV based on published JCV sequences, as illustrated in Fig. 1. PCR may be run using well-known condictions, e.g., a 50-µl volumes by using the "hot start" technique (with Ampliwax, Perkin-Elmer), with 50 pmol of each primer, 1-2.5 units of recombinant Taq polymerase (GIBCO), a standard buffer (50 mM KCl/10 mM Tris·HCl/2 mM MgCl2), 200 µM of each dNTP, and 500-1,000 ng of template DNA. The initial denaturation is at 94°C for 3 min and a final extension of 72°C for 10 min. Oligonucleotides used as PCR primers and the positions described below correspond to the positions on the Mad1 isolate. For example, a 520-bp target is amplified by using the primers JCT1 (5'-AATAGTGGTTTACCTTAAAG, complementary to positions 4481-4500 on the sense strand) and JCT2 (5'-TGAATAGGGAGGAATCCATG, complementary to positions 5000-4981 on the antisense strand), by using 1.5 mM MgC12 in this reaction, for 40 cycles at 92°C for 30 sec, 50°C for 30 sec, and 72°C for 30 sec. PCR products are electrophoresed on agarose gels and isolated for cloning. Alternatively, after 40 cycles of PCR with the JCT1/JCT2 primers, nested PCR was performed by using 1-2% of the first-round product for another 30 cycles, at 62°C for 1 min for the annealing/extension reaction, with primers 90PRO and ESPB (5'-CTGCATGGGGGAACATTCCTGTC, corresponding to positions 4949-4927). This procedure amplifies a 429-bp target sequence. Similarly, by using primers FPO and ESPB, a 229-bp amplicon is generated.

JCV in Cultured Human Colorectal Cancer Cell Lines. Colon cancer cell lines SW480, HCT116, HT29, and LoVo were obtained from the American Type Culture Collection (ATCC), grown under standard conditions as recommended by ATCC. The DNA was extracted and JCV sequences were sought by PCR, as described above.

Cloning and Sequencing of JCV DNA. PCR products may be cloned into pUC18 (SureClone, Amersham Pharmacia), Bluescript (Stratagene) or other vector system according to the manufacturer's instructions, grown in INVα (Invitrogen), and reamplified, and the PCR products were sequenced from both ends of the PCR products by using original and nested primers with an ABI 310 Genetic Analyzer (Perkin-Elmer). Sequencing of the regulatory region downstream of the origin of replication was performed to further confirm the identity of the virus as distinct from BK virus or SV40, by using PCR primers spanning the T antigen (5060-5079) and regulatory region (297-317). Clones were derived from the amplified bands of selected samples in a Bluescript vector and sequenced.

Figure 1 is a map of the Mad1 JCV, which may be used for making constructs, vectors, for use in polymerase chain reaction and general cloning of genes or portions thereof. Those of skill in the art will recognize that splicing using restriction enzymes, linkers and PCR oligonucleotides with restriction enzymes sites and even blunt end or partially blunt end ligation may be used to insert the gene (or fragment thereof) or interest into a vector may be achieved based design requirements.

### Example 2. Vaccinia-based Oral Vaccine.

The cloned JCV may be used in conjunction with, e.g., U.S. Patent No. 6,960,345, issued to Moyer, which teaches oral vaccinia-based formulations. The present invention may be used in conjunction with portions of the compositions and methods taught therein to deliver immunogenic JCV proteins as part of an oral vaccinia-based formulation, relevant portions incorporated herein by reference. Briefly, one or more genes and/or portions of the JCV genome (including, e.g., multiple strains of JCV) are spliced into the vaccinia genome using methods well-known to those of skill in the art. Replication defective vaccinia is particularly useful for use with the present invention to serve as the carrier and/or as an adjuvant for JCV antigen presentation and/or processing. Therefore, JCV is used in conjunction with the methods and systems of Moyer for generating a safe and effective oral smallpox vaccine for humans using a genetically defective strain of vaccinia virus to confer immunity following oral delivery of the vaccine. JCV antigens are presented along during vaccinia virus propagation developed for gene therapy applications, and pharmaceuticals and nutraceuticals packaging and formulation technologies.

The JCV vaccine may be delivered as a live virus with the ability to express JCV viral proteins but unable to achieve complete, lytic virus replication, or it may be derived from such a virus, contain additional immunogens, or be delivered as viral antigens. The JCV-vaccinia vaccine may be used for, e.g., preclinical testing of the vaccine invention for safety, efficacy and potency with the use of human intestinal and other test cells and diagnostic test systems and kits.

Vaccinia-Based Oral Immunization and Methods. A variety of oral immunization formulae can be used for immunization. Oral immunization is done by preparing a formula in which the vaccinia virus that serves as the carrier for one or more JCV genes and/or portions thereof, remains viable (as determined by infectivity of released virus from the orally delivered paste and separate components of the paste formulae listed below) and is captured in nanoparticles and micelles as part of the protective formulation that includes aqueous and oil-based components, as well as suspending agents and carriers that protect the virus from degradation and allow it to be absorbed from the oral cavity and the intestine.

As an example of the formulation, virus is prepared by mixing virus in a solution of Hetastarch (hydroxyethyl starch, clinical grade; 6% w/v; Baxter), 40% (v/v) mannitol [UPS grade higher; SIGMA or other vendor], 5% (v/v) glycerol (UPS grade; SIGMA or other vendor), 0.5% (w/v) gelatin (SIGMA) at a volume that will achieve a final concentration of 10⁵ to 10⁸ infectious units per ml, depending on the effective or test dose expected (e.g., 10⁶ to 10⁸ for humans, depending on immunization status). Gel-sol virus carrier (GSVC) excipient components were prepared as an equal mixture (1:1:1; Avicel® CE-15 (microcrystalline cellulose and guar gum), Avicel® 591 (water-dispersible microcrystalline cellulose containing sodium carboxymethylcellulose (NaCMC) and Ac-Di-Sol® (internally-crosslinked, water insoluble sodium carboxymethylcellulose (NaCMC))(FMC Products) which was slowly added (with vortexing) to a final concentration of 10% (w/v).

Taste-testing is used to select combinations of flavors and excipients that are palatable with little or no aftertaste and good mouth-feel. The vaccinia carrier virus may be stored in dry or liquid form, making sure that infectious virus stability is analyzed and optimized as will be known to those of skill in the art.

Bioassays and Biochemical Methods for Safety, Efficacy and Potency. A variety of bioassays and biochemical analyses are done to evaluate the vaccine. These include: (a) human cell line nonpermissiveness with expression of vaccine antigens (a safety test); (b) viral antigen expression and production compared to previous lots and reference standards (i.e., potency); and (c) activation of humoral and cell-mediated immunity (e.g., potency and efficacy) in infected animals. These are imperative types of assays to evaluate each virus lot and the overall potential variability between lots of virus.

### Example 2. Attenuated Polio Virus Carrier/vector for JCV.

Attenuated polio virus type 1 strain: PV1/Sabin may be modified to include one or more genes from one or more JCV strains and grown in Hep-2c human epithelial cells originating from an epidermoid carcinoma of the larynx. On day 1, the cells are placed in culture. For example, the cells and virus may be grown in a suspension of 2.5 x 10⁵ cells/ml in a MEM medium, 10% fetal calf serum (FCS), 0.5% gentamycin, and seeded with 200 microliters/well (i.e., 5 x 10⁴ cells/well). The cells were incubated at 37° C in the presence of 5% CO₂.

Dilutions of viral suspensions are prepared, of 10 in 10 up to 10⁻⁴, then dilutions of 4 in 4 up to 10⁻⁸ in MEM medium, with no FCS, 0.5% gentamycin (50 microliters/well and 4 wells/dilution). For use during infection, attenuated polio virus-JCV is diluted in MEM medium, 3% FCS, 0.5% gentamycin so as to obtain a final concentration of 25 micrograms/ml of peptide (knowing that for the test, 150 microliters of medium containing the peptide was added to 50 microliters of viral dilution).

The infection step may be carried out by emptying the wells with the target/host cells (by aspiration) then adding the following elements: 150 microliters/well of diluted attenuated polio virus-JCV or medium for the untreated plate; 50 microliters /well of 10⁻⁵ to 10⁸ viral dilutions, 4 wells/dilution. The host cells and the attenuated polio virus-JCV are incubated at 37°C for 5 days in the presence of 5% of CO₂. 5 days after infection, the CPD50/ml titers are determined.

It is contemplated that any embodiment discussed in this specification can be implemented with respect to any method, kit, reagent, or composition of the invention, and vice versa. Furthermore, compositions of the invention can be used to achieve methods of the invention. It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof' as used herein refers to all permutations and combinations of the listed items preceding the term. For example, "A, B, C, or combinations thereof' is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. All such similar substitutes and modifications apparent to those skilled in the art are deemed to be within the spirit, scope and concept of the invention as defined by the appended claims.

Preferably the present invention is defined as in the following definitions:
1. A vaccine comprising:
   one or more JC virus antigens in a carrier adapted to trigger a JC virus-specific immune response in the human intestinal tract.
2. The vaccine of definition 1, wherein the one or more JC virus antigens are obtained from a virus that has been heat-killed, attenuated, chemically-inactivated, mechanically inactivated or combinations thereof.
3. The vaccine of definition 1 or 2, wherein the one or more JC virus antigens are selected from one or more strains of JC virus.
4. The vaccine of any one of definition 1 to 3, wherein the one or more JC virus antigens comprise one or more recombinant proteins, synthetic peptides or fragments of JC virus.
5. The vaccine of definition 1, 3 or 4, wherein the one or more JC virus antigens are produced expressed in a cell from an expression vector that comprises one or more genes or gene fragments from one or more strains of the JC virus.
6. The vaccine of any one of definition 1 to 5, wherein the one or more JC virus antigens are selected to trigger a cytotoxic T-cell immune response, a humoral immune response or a combination thereof.
7. The vaccine of any one of definition 1 to 6, wherein the one or more JC virus antigens are inserted for expression in a carrier virus.
8. The vaccine of any one of definition 1 to 7, wherein the one or more JC virus antigens are inserted as gene or gene fragments that are expressed in a carrier virus comprising an attenuated poliovirus.
9. The vaccine of any one of definition 1 to 8, wherein the carrier comprises a pharmaceutically acceptable salt.
10. The vaccine of any one of definition 1 to 9, wherein the carrier comprises an adjuvant selected from CFA, IFA, alum, a carrier virus, high molecular weight polysaccharides, glycoproteins and combinations thereof.
11. The vaccine of any one of definition 1 to 10, wherein the one or more JC virus antigens and the carrier are lyophilized, vacuum-dried, vacuum heat-dried, freeze-sprayed or combinations thereof.
12. The vaccine of any one of definition 1 to 11, wherein the carrier comprises an excipient, an adjuvant, an absorption enhancer, a release-rate controlling polymer, a stability enhancer, or combinations thereof.
13. The vaccine of definition 1, wherein the carrier comprises an attenuated and/or inactivated polio virus selected from the group consisting of polio virus type 1, polio virus type 2, polio virus type 3, and mixtures thereof, comprising one or more JC virus genes.
14. The vaccine of definition 1, wherein the carrier comprises an attenuated poliovirus, inactivated poliovirus, hepatitis A virus, echovirus, rhinovirus and coxsackievirus, comprising one or more JC virus genes.
15. A vaccine of any one of definition 1 to 14 for vaccinating a mammal against JC Virus.
16. The vaccine of definition 15, wherein the one or more JC virus antigens are inserted for expression in a carrier virus selected from an attenuated poliovirus, a heat-killed poliovirus, a vaccinia virus, influenza virus, Japanese encephalitis virus, yellow fever virus, measles virus, rubella virus, mumps virus, hepatitis B virus, adenovirus, Epstein-Barr virus, distemper virus, rabies virus, Sendai virus and Newcastle disease virus.
17. Use of one or more JC virus antigens in a carrier adapted to trigger a JC virus-specific immune response in the human intestinal tract and as defined in any one of definiton 1 to 14 for the manufacture of a vaccine for vaccinating a mammal against JC virus.

### REFERENCES

(1) Laghi L, Randolph AE, Chauhan DP, Marra G, Major EO, Neel JV et al. JC virus DNA is present in the mucosa of the human colon and in colorectal cancers. Proc Natl Acad Sci U S A 1999; 96(13):7484-7489.
(2) Ricciardiello L, Laghi L, Ramamirtham P, Chang CL, Chang DK, Randolph AE et al. JC virus DNA sequences are frequently present in the human upper and lower gastrointestinal tract. Gastroenterology 2000; 119(5):1228-1235.
(3) Ricciardiello L, Chang DK, Laghi L, Goel A, Chang CL, Boland CR. Mad-1 is the exclusive JC virus strain present in the human colon, and its transcriptional control region has a deleted 98-base-pair sequence in colon cancer tissues. J Virol 2001; 75(4):1996-2001.
(4) Ricciardiello L, Baglioni M, Giovannini C, Pariali M, Cenacchi G, Ripalti A et al. Induction of chromosomal instability in colonic cells by the human polyomavirus JC virus. Cancer Res 2003; 63(21):7256-7262.
(5) Del Valle L, White MK, Enam S, Oviedo SP, Bromer MQ, Thomas RM et al. Detection of JC virus DNA sequences and expression of viral T antigen and agnoprotein in esophageal carcinoma. Cancer 2005; 103(3):516-527.
(6) Hamilton RS, Gravell M, Major EO. Comparison of antibody titers determined by hemagglutination inhibition and enzyme immunoassay for JC virus and BK virus. J Clin Microbiol 2000; 38(1):105-109.

## Claims

1. An oral vaccine comprising one or more JC virus antigens in a replication-defective vaccinia carrier, wherein the JC antigens are suitable for inducing a immune response against the JS virus in a mammal's intestinal tract.

2. Vaccine of claim 1, wherein the one or more JC virus antigens are obtained from a virus that has been heat-killed, attenuated, chemically-inactivated, mechanically inactivated or combinations thereof.

3. Vaccine of claim 1 or 2, wherein the one or more JC virus antigens are selected from one or more strains of JC virus.

4. Vaccine of any one of claims 1 to 3, wherein the one or more JC virus antigens comprise one or more recombinant proteins, or fragments of JC virus.

5. Vaccine of claim 1, 3 or 4, wherein the one or more JC virus antigens are produced in a cell from an expression vector that comprises one or more genes or gene fragments from one or more strains of the JC virus.

6. Vaccine of any one of claims 1 to 5, wherein the one or more JC virus antigens are selected to trigger a cytotoxic T-cell immune response, a humoral immune response or a combination thereof.

7. Vaccine of any one of claims 1 to 6, comprising a pharmaceutically acceptable salt.

8. Vaccine of any one of claims 1 to 7, comprising an adjuvant selected from CFA, IFA, alum, a carrier virus, high molecular weight polysaccharides, glycoproteins and combinations thereof.

9. Vaccine of any one of claims 1 to 8, wherein the vaccinia-based vaccine comprises one or more portions of the JC virus genome spliced into the vaccinia genome.

10. Vaccine of any one of claims 1 to 9, comprising an excipient, an adjuvant, an absorption enhancer, a release-rate controlling polymer, a stability enhancer, or combinations thereof.

11. Vaccine of any one of claims 1 to 10 for use in vaccinating a mammal against JC virus.
